# EUROPEAN PATENT SPECIFICATION

(11) **EP 3 074 052 B1**
(45) Date of publication and mention of the grant of the patent: **09.05.2018**
(21) Application number: 14806230.0
(22) Date of filing: 28.11.2014
(51) Int. Cl.: A61L 2/07, A61L 2/26, B01J 3/04

(54) **VACUUM SYSTEM FOR STERILISATION EQUIPMENT WITHOUT THE USE OF WATER AND NOT LIMITED TO SATURATED STEAM**
VAKUUMSYSTEM FÜR STERILISATIONSVORRICHTUNG OHNE VERWENDUNG VON WASSER UND NICHT AUSSCHLIESSLICH FÜR SATTDAMPF
SYSTÈME SOUS VIDE POUR UN ÉQUIPEMENT DE STÉRILISATION SANS UTILISER D'EAU ET NON LIMITÉ À LA VAPEUR SATURÉE

(30) Priority: 28.11.2013 BR 102013307025
(43) Date of publication of application: 05.10.2016
(73) Proprietor: CISA Production S.r.l., 55100 Lucca (LU) (IT)
(72) Inventor: BARBOSA RODRIGUES, Sandoval, 89237-322 Joinville - Santa Catarina (BR)
(74) Representative: Trillat, Anne-Cecile
(86) International application number: PCT/EP2014/075913
(87) International publication number: WO 2015/079013

(56) References cited:
- DE-A1- 19 751 692
- JP-A- 2001 029 437
- US-A1- 2009 123 341
- US-A1- 2011 076 192

## Description

### Technical Field

The present invention relates to a vacuum system for sterilisation equipment without the use of water not limited to saturated steam, particularly for use in equipment for sterilising medical implements for clinical, hospital or similar use. The vacuum system for sterilisation equipment comprises a dry vacuum pump not limited to saturated steam, said pump being connected to peripheral equipment such as a PME (Gas Ballast) valve for effecting the removal of waste water from the inside of the pump in certain phases of the cycle in which a vacuum is not required.

### Background Art

Vacuum systems for sterilisation equipment are known, in which the vacuum is generated by means of a liquid ring pump, these systems requiring a high water consumption, approximately 300 to 500 litres per sterilisation cycle. Alternatively, the vacuum in vacuum system for sterilisation equipments can be provided by means of an oil-lubricated rotary vane pump. However, this system is limited to saturated steam and necessitates the use of many valves to limit the condensation, thereby increasing the risk of faults in the system. An additional drawback is that the maintaining costs are very high due to the necessity of several spare parts for the pump and the expected maintenance of the oil and the filters. Document US A 2009/123341 describes a system comprising a rotary vane vacuum pump operating in saturated or almost saturated steam conditions at high temperatures, without a cooling system, and which comprises a lubrication circuit with a tank in which circulates a lubricating fluid . The tank is able to withstand high temperatures and has a high anti-emulsifying capacity, with a filtration means capable of separating the steam from the lubricating fluid. According to this document, a typical process of sterilization not using mains water as an exchange fluid for the cooling system, comprises the steps of conditioning, heating, sterilization, chamber discharge, drying and aeration.

In particular, the phase of conditioning serves to remove the air inside the sterilization chamber by means of a vacuum pump coupled to the sterilization chamber; the lower the pressure reached during this phase, the better the heat distribution during sterilization.

The heating phase brings the temperature of the chamber, and consequently of the load inside it, to the set value established for the sterilization.

In the sterilization phase, the load inside the chamber is maintained at the temperature established for the time necessary for abatement of the bacteria count.

In the chamber discharge phase, the steam is removed from the chamber up to atmospheric pressure.

In the drying phase, a vacuum is maintained for a pre-set time inside the chamber to allow evaporation of the condensation from the load.

Finally, in the aeration phase, sterile air is fed to balance the pressure in the chamber for subsequent opening. Although these vacuum systems for sterilisation equipment perform the function of generating vacuum, these systems do not guarantee high efficiency of the pump during vacuum generation.

In the case of the vacuum system for sterilisation equipment, in which the vacuum is generated by means of a liquid ring pump, further disadvantages are that this system has a high water consumption per sterilisation cycle. Furthermore, the system efficiency is directly linked to the water temperature insofar as the higher the temperature the more steam will be present inside the sterilisation chamber, thereby making vacuum generation difficult.

With regard to the system of document US A 2009/123341 although the pump generates the vacuum in saturated, or almost saturated steam conditions at high temperatures, this system also uses a large number of valves to limit the condensation, thus increasing the cost of maintenance. To solve the problem of high water consumption, vacuum generating systems are known not using water. However, the fact that these systems do not use water, does not mean that the formation of steam during vacuum generation is prevented. In fact, the steam generated usually returns to the pump, thereby reducing its efficiency during vacuum generation.

It is therefore necessary to develop vacuum systems for sterilisation equipment without the use of water and not limited to saturated steam, without any loss in efficiency of the pump in generating vacuum during the sterilisation, for example of medical instruments for clinical, hospital or similar use.

### Disclosure of Invention

For the purpose of eliminating these drawbacks the present invention proposes a vacuum system for sterilisation equipment in which the system comprises a dry vacuum pump connected to a valve, such as a PME valve for removing the waste water from inside the pump in certain phases of the sterilisation cycle in which the vacuum is not required, a curved duct or pipe at the pump inlet to enable the condensation to be purged by a thermostatic valve and a check valve installed in the pump outlet to prevent the return of condensation.

A further objective of the present invention is to provide a vacuum system for sterilisation equipment, without the use of water to generate the vacuum, so that it does not depend on the temperature to improve its efficiency, thus reducing water consumption to zero.
A further objective of the present invention is to provide a vacuum system which is not limited to saturated steam, thus enabling all the steam in the system to pass through the pump without damaging or negatively influencing the sterilisation process

Advantageously the present invention presents a vacuum system for sterilisation equipment without the use of water not limited to saturated steam, which guarantees greater reliability and security during the sterilisation processes, thus increasing the efficiency of the dry vacuum pump.

The present invention describes a vacuum system for sterilisation equipment without the use of water and not limited to saturated steam, thus increasing the efficiency in generating a vacuum.

The vacuum system comprises a dry vacuum pump connected to a valve, such as a PME valve, for removing the waste water inside the pump in certain phases of the sterilisation cycle in which the vacuum is not required, a curved duct or pipe at the pump inlet to enable the condensation to be purged by a thermostatic valve and a check valve installed in the pump outlet to prevent the return of condensation.

Schematic figures of a particular embodiment of the invention are presented below, in which the dimensions and proportions are not necessarily the actual values since the sole purpose of the figures is to present in a didactic manner the various aspects of the system, the extent of protection of which is determined exclusively by the scope of the annexed claims.

### Brief Description of Drawings

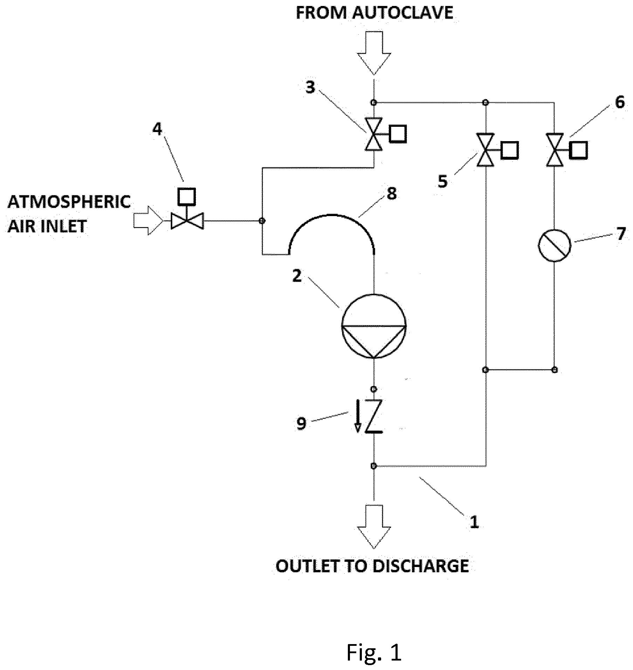

The invention will be described with reference to the annexed figure, in which Figure 1 illustrates a functional diagram of the vacuum system for sterilisation equipment.

### Mode for Carrying Out the Invention

Figure 1 illustrates the vacuum system (1) of the present invention for creating vacuum in a sterilization chamber (not shown in the figure) of a sterilization equipment, wherein the vacuum system (1) comprises an inlet connected to an outlet of the sterilization chamber and a discharge outlet. As illustrated in Figure 1, the vacuum system (1) for sterilisation equipment without the use of water, and not limited to saturated steam, is formed by a vacuum pump (2) having an inlet and an outlet coupled to the discharge outlet of the vacuum system (1) and a set of valves (3, 4) positioned between the outlet of the sterilization chamber (and therefore the inlet of the vacuum system (1)) and the discharge outlet of the vacuum system (1) and connected to the inlet of the vacuum pump (2). The set of valves (3, 4, 5, 6) comprises an inlet valve (3) connected to the inlet of the vacuum system (1), an atmospheric air inlet valve (4) coupled to the inlet valve (3) for introducing atmospheric air into the vacuum system (1), an atmospheric pressure stabilization valve (5) positioned between the atmospheric air inlet valve (4) and the discharge outlet of the vacuum system (1) and a discharge valve (6) connected in series to a thermostatic condensation discharge valve (7). The discharge valve (6) and the thermostatic condensation discharge valve (7) are connected in parallel to the atmospheric pressure stabilization valve (5) and are positioned between the atmospheric air inlet valve (4) and the discharge outlet of the vacuum system (1). In particular, the set of valves (3, 4) is connected to the inlet of the vacuum pump (2) through a curved duct (8) to prevent condensation from entering in the vacuum pump (2).

According to a preferred configuration, the the curved duct (8) is formed by a semicircular section joining two parallel vertical sections connected to the inlet of the vacuum pump (2) and to the set of valves (3, 4), respectively.

The particular connection among the components forming the vacuum system (1) imparts to the same greater efficiency during the generation of the vacuum during the sterilisation of medical implements for clinical, hospital or similar use. In order to increase the efficiency of the vacuum pump (2) during the actuation of the sterilisation equipment, the atmospheric air inlet valve (4) is actuated, thereby allowing the admission of atmospheric air for cleaning the vacuum system (1), preferably during the phases in which vacuum is not required in the system (1). However, the atmospheric air inlet valve (4) may also be actuated during the vacuum generating phases to clean the vacuum system (1). In particular, the atmospheric air inlet valve (4) is positioned between the inlet valve (3) and the rest of the vacuum system (1), specifically the inlet of the vacuum pump (2), the atmospheric pressure stabilisation valve (5) and the discharge valve (6).

When the atmospheric air inlet valve (4) is actuated it releases air to the vacuum pump (2), causing the condensation generated to be purged out of the pump and preventing the condensation from accumulating in the pump (2) and affecting its performance.

In a particular embodiment of the present invention, the inlet valve (3), which connects the vacuum system (1) to the sterilisation chamber, is electrically and/or pneumatically coupled to the atmospheric air inlet valve (4), which admits external air for cleaning when vacuum is not required in the system (1).The atmospheric air inlet valve (4) is connected to the atmospheric pressure stabilisation valve (5), serving as a bypass for the pump (2) when there is pressure in the sterilisation chamber, in order to avoid excess pressure in the same. The discharge valve (6) is connected in series to the thermostatic valve (7) in order to give access to said thermostatic condensation discharge valve (7)
Valve (7) serves for removing condensation in the conditioning and heating phase. These two phases are parts of a typical sterilization cycle known in the art, composed by following stages inside autoclave chamber:
- Vacuum test
- Steam inlet
- Conditioning
- Heating
- Sterilization
- Cooling
- Washing
- Drying
- Aeration.

The set of valves (3, 4) is connected to the inlet of the vacuum pump (2) by a duct creating a bend or curved connection (8) of sufficient height to prevent the condensate from entering the vacuum pump (2) by gravity.

With regard to the atmospheric pressure stabilization valve (5), the thermostatic condensation discharge valve (7) and the vacuum pump (2), their outlets are connected. Thus, all the condensation generated due to the sterilisation is directed to the discharge outlet of the vacuum system (1).

Optionally, the outlet of the vacuum pump (2) may be connected to a check valve (9), thereby preventing the return of the condensation inside the pump (2) deriving from the atmospheric pressure stabilization valve (5) and the thermostatic condensation discharge valve (7), or eventually from other equipments connected to the vacuum system (1). The atmospheric air inlet valve (4) may also comprise a muffler filter (not illustrated in the figure) for the purpose of reducing the noise generated during the operation of this valve (4).

Advantageously, since the vacuum system (1) does not require water to generate the vacuum, it does not therefore depend on the temperature of the water to generate a vacuum. The system (1) therefore provides optimum repeatability of the process independently of the season of the year, for example, where the variation in the temperature of the water may alter the performance of the pump (2).

The person skilled in the art will quickly realise, from the description and the flow chart presented, that there are various methods of implementing the invention without departing from the scope of the annexed claims.

## Claims

1. A vacuum system (1) for creating vacuum in a sterilization chamber of a sterilization equipment, the vacuum system (1) having an inlet connected to an outlet of the sterilization chamber and a discharge outlet, the vacuum system (1) comprising:
a vacuum pump (2) having an inlet and an outlet coupled to the discharge outlet of the vacuum system (1) and
a set of valves (3, 4, 5, 6) positioned between the outlet of the sterilization chamber and the discharge outlet of the vacuum system (1) and,
wherein the set of valves (3, 4, 5, 6) comprises an inlet valve (3) connected to the inlet of the vacuum system (1), an atmospheric air inlet valve (4) coupled to the inlet valve (3) for introducing atmospheric air into the vacuum system (1), an atmospheric pressure stabilization valve (5) positioned between the atmospheric air inlet valve (4) and the discharge outlet of the vacuum system (1) and a discharge valve (6) connected in series to a thermostatic condensation discharge valve (7), the discharge valve (6) and the thermostatic condensation discharge valve (7) being connected in parallel to the atmospheric pressure stabilization valve (5) between the atmospheric air inlet valve (4) and the discharge outlet of the vacuum system (1), and
wherein the set of valves (3, 4) is connected to the inlet of the vacuum pump (2) through a duct (8) able to prevent condensation from entering in the vacuum pump (2).

2. The vacuum system (1) according to claim 1, wherein the duct (8) is curved.

3. The vacuum system (1) according to claim 1 or 2, wherein the duct (8) is formed by a semicircular section joining two parallel vertical sections connected to the inlet of the vacuum pump (2) and to the set of valves (3, 4), respectively.

4. The vacuum system (1) according to one of the preceding claims, wherein the inlet valve (3) is electrically coupled to the atmospheric air inlet valve (4).

5. The vacuum system (1) according to one of the claims 1, 2 or 3, wherein the inlet valve (3) is pneumatically coupled to the atmospheric air inlet valve (4).

6. The vacuum system (1) according to one of the preceding claims, further comprising a check valve (9) positioned between the outlet of the vacuum pump (2) and the discharge outlet of the vacuum system (1).

7. The vacuum system (1) according to one of the preceding claims, wherein the atmospheric air inlet valve (4) comprises a muffler filter.

8. The vacuum system (1) according to one of the preceding claims, wherein the vacuum pump (2) is a dry vacuum pump.

## Patentansprüche

1. Vakuumsystem (1) zum Erzeugen eines Vakuums in einer Sterilisationskammer einer Sterilisationsausrüstung, wobei das Vakuumsystem (1) einen Einlass, der mit einem Auslass der Sterilisationskammer verbunden ist, und einen Austragauslass aufweist, wobei das Vakuumsystem (1) umfasst:
eine Vakuumpumpe (2) mit einem Einlass und einem Auslass, der mit dem Austragauslass des Vakuumsystems (1) gekoppelt ist, und
eine Reihe von Ventilen (3, 4, 5, 6), die zwischen dem Auslass der Sterilisationskammer und dem Austragauslass des Vakuumsystems (1) positioniert sind, und
wobei die Reihe von Ventilen (3, 4, 5, 6) ein Einlassventil (3), das mit dem Einlass des Vakuumsystems (1) verbunden ist, ein Atmosphärenlufteinlassventil (4), das mit dem Einlassventil (3) gekoppelt ist, um Atmosphärenluft in das Vakuumsystem (1) einzuführen, ein Atmosphärendruckstabilisierungsventil (5), das zwischen dem Atmosphärenlufteinlassventil (4) und dem Austragauslass des Vakuumsystems (1) positioniert ist, und ein Austragventil (6), das mit einem thermostatischen Kondensataustragventil (7) in Reihe verbunden ist, umfasst, wobei das Austragventil (6) und das thermostatische Kondensataustragventil (7) mit dem Atmosphärendruckstabilisierungsventil (5) zwischen dem Atmosphärenlufteinlassventil (4) und dem Austragauslass des Vakuumsystems (1) parallel verbunden sind, und
wobei die Reihe von Ventilen (3, 4) mit dem Einlass der Vakuumpumpe (2) durch einen Kanal (8) verbunden ist, der verhindern kann, dass Kondensat in die Vakuumpumpe (2) eindringt.

2. Vakuumsystem (1) nach Anspruch 1, wobei der Kanal (8) gekrümmt ist.

3. Vakuumsystem (1) nach Anspruch 1 oder 2, wobei der Kanal (8) durch einen halbkreisförmigen Abschnitt gebildet ist, der zwei parallele vertikale Abschnitte verbindet, die entsprechend mit dem Einlass der Vakuumpumpe (2) und der Reihe von Ventilen (3, 4) verbunden sind.

4. Vakuumsystem (1) nach einem der vorstehenden Ansprüche, wobei das Einlassventil (3) mit dem Atmosphärenlufteinlassventil (4) elektrisch gekoppelt ist.

5. Vakuumsystem (1) nach einem der Ansprüche 1, 2 oder 3, wobei das Einlassventil (3) mit dem Atmosphärenlufteinlassventil (4) pneumatisch gekoppelt ist.

6. Vakuumsystem (1) nach einem der vorstehenden Ansprüche, weiter umfassend ein Rückschlagventil (9), das zwischen dem Auslass der Vakuumpumpe (2) und dem Austragauslass des Vakuumsystems (1) positioniert ist.

7. Vakuumsystem (1) nach einem der vorstehenden Ansprüche, wobei das Atmosphärenlufteinlassventil (4) einen Schalldämpferfilter umfasst.

8. Vakuumsystem (1) nach einem der vorstehenden Ansprüche, wobei die Vakuumpumpe (2) eine Trockenvakuumpumpe ist.

## Revendications

1. Système à vide (1) pour créer un vide dans une chambre de stérilisation d'un équipement de stérilisation, le système à vide (1) ayant une entrée connectée à une sortie de la chambre de stérilisation et une sortie de décharge, le système à vide (1) comprenant :
une pompe à vide (2) ayant une entrée et une sortie couplée à la sortie de décharge du système à vide (1), et
un ensemble de soupapes (3, 4, 5, 6) positionnées entre la sortie de la chambre de stérilisation et la sortie de décharge du système à vide (1), et
dans lequel l'ensemble de soupapes (3, 4, 5, 6) comprend une soupape d'entrée (3) connectée à l'entrée du système à vide (1), une soupape d'entrée d'air atmosphérique (4) couplée à la soupape d'entrée (3) pour introduire de l'air atmosphérique dans le système à vide (1), une soupape de stabilisation de pression atmosphérique (5) positionnée entre la soupape d'entrée d'air atmosphérique (4) et la sortie de décharge du système à vide (1) et une soupape de décharge (6) connectée en série à une soupape de décharge de condensation thermostatique (7), la soupape de décharge (6) et la soupape de décharge de condensation thermostatique (7) étant connectées en parallèle à la soupape de stabilisation de pression atmosphérique (5) entre la soupape d'entrée d'air atmosphérique (4) et la sortie de décharge du système à vide (1), et
dans lequel l'ensemble de soupapes (3, 4) est connecté à l'entrée de la pompe à vide (2) via un conduit (8) capable d'empêcher de la condensation d'entrer dans la pompe à vide (2).

2. Système à vide (1) selon la revendication 1, dans lequel le conduit (8) est incurvé.

3. Système à vide (1) selon la revendication 1 ou 2, dans lequel le conduit (8) est formé par une section semi-circulaire reliant deux sections verticales parallèles connectées respectivement à l'entrée de la pompe à vide (2) et à l'ensemble de soupapes (3, 4).

4. Système à vide (1) selon l'une des revendications précédentes, dans lequel la soupape d'entrée (3) est couplée électriquement à la soupape d'entrée d'air atmosphérique (4).

5. Système à vide (1) selon l'une des revendications 1, 2 ou 3, dans lequel la soupape d'entrée (3) est couplée pneumatiquement à la soupape d'entrée d'air atmosphérique (4).

6. Système à vide (1) selon l'une des revendications précédentes, comprenant en outre une soupape anti-retour (9) positionnée entre la sortie de la pompe à vide (2) et la sortie de décharge du système à vide (1) .

7. Système à vide (1) selon l'une des revendications précédentes, dans lequel la soupape d'entrée d'air atmosphérique (4) comprend un filtre de silencieux.

8. Système à vide (1) selon l'une des revendications précédentes, dans lequel la pompe à vide (2) est une pompe à vide à sec.
